Europäisches Patentamt

European Patent Office

Office européen des brevets

(19)

(11) Publication number: **0 448 878 A2**

# EUROPEAN PATENT APPLICATION

(21) Application number: 90314025.9

(51) Int. Cl.5: **A61M 16/04**

(22) Date of filing: **20.12.90**

(30) Priority: **27.02.90 GB 9004315**

(43) Date of publication of application:
**02.10.91 Bulletin 91/40**

(84) Designated Contracting States:
**AT BE CH DE DK ES FR GB GR IT LI LU NL SE**

(71) Applicant: **Brain, Archibald Ian Jeremy, Dr.**
**10 Preston Drive**
**Wanstead London E11(GB)**

(72) Inventor: **Brain, Archibald Ian Jeremy, Dr.**
**10 Preston Drive**
**Wanstead London E11(GB)**

(74) Representative: **Driver, Virginia Rozanne et al**
**Page White & Farrer 54 Doughty Street**
**London WC1N 2LS(GB)**

(54) **Artificial airway device.**

(57) An artificial airway device to facilitate lung ventilation in an unconscious patient, comprises a mask (12) having a flexible annular peripheral formation of roughly elliptical shape capable of conforming to, and of readily fitting within, the actual and potential space behind the larynx so as to form a seal around the circumference of the laryngeal inlet without the device penetrating into the interior of the larynx, the annular peripheral formation surrounding a hollow interior space or lumen (18) of the mask into which opens an airway conduit (22) whose length is of the same order as that of the mask. An airway tube (10) is connectable to the airway conduit (22) via a detachable connector (51), so that the mask can be inserted into operative position in a patient via the mouth while the airway tube can subsequently be inserted through the nose and connected to the airway conduit by the detachable connecting means.

*FIG.5*

EP 0 448 878 A2

This invention relates to artificial airway devices to facilitate lung ventilation in unconscious patients, and more specifically to such devices designed for placing in the oropharynx of the patient in order to prevent airway obstruction and to permit either spontaneous or controlled ventilation.

To maintain the airway of an unconscious patient, and to achieve the objectives mentioned above, it is normal practice in general anaesthesia to use an endotracheal tube, which is a flexible tube of rubber or plastics, usually with an inflatable cuff around the distal end. Alternatively, an oro- or naso-pharyngeal airway may be used, which is a flexible tube extending from the mouth or nose into the pharynx but not into the larynx, and which is used in conjunction with a face mask, unlike the endotracheal tube. While preventing obstruction of the airway by the tongue, the oro- or naso-pharyngeal airway cannot conveniently be used for controlled ventilation and does not prevent inhalation of extraneous matter.

The endotracheal tube is introduced through the larynx into the trachea or windpipe, whereupon the cuff is inflated through a small auxiliary tube to seal against the wall of the trachea. Introduction of the endotracheal tube whether through the mouth (orotracheal intubation) or through the nose (nasal intubation) is a skilled operation normally requiring use of a laryngoscope to guide the tube through the larynx, past the vocal cords and into the trachea. There is a risk that the tube or the laryngoscope may cause damage to soft tissues or to the sensitive structures of the larynx. It is not always possible to see the larynx, making intubation difficult or impossible in some patients. There can be a risk of accidental intubation of the oesophagus or of the right or left main bronchus. Placing of the tube in the trachea effectively narrows the interior passage or lumen of the trachea and provides a potential source of damage through infection or pressure while preventing normal upward flow of mucus from the trachea and rendering effective coughing impossible.

Nasal intubation is used to denote the introduction of a flexible tube for airway management via the nasal passages of a patient. This is used in operations where a surgeon requires access to the oral cavity and/or throat. A common example is dental surgery. The technique involves paralysing the patient by giving a neuromuscular paralysing drug to enable the laryngeal inlet to be penetrated without the patient being able to react. A small gauge nasal tube is then inserted through the wider of the two nostrils, down into the back of the throat and, with or without the aid of a guiding forceps and laryngoscope, through the vocal cords and into the trachea.

This is a more difficult technique than orotracheal intubation (via the mouth), particularly if it is performed blindly.

In my British Patent Specification No. 2111394B, I have described and claimed an artificial airway device comprising a curved or flexible tube and a mask portion secured to one end of the tube, the mask portion having a flexible annular peripheral formation which may be inflatable and which surrounds a hollow interior space or lumen of the mask portion, said annular peripheral formation of the mask portion being pre-formed with a roughly elliptical shape such as to be capable of conforming to, and of fitting readily within, the actual and potential space behind the larynx so as to form a seal around the circumference of the laryngeal inlet without the device penetrating into the interior of the larynx, the tube opening into the lumen of the mask portion to provide the airway with the axis of the tube substantially aligned with the length of the roughly elliptical annular peripheral formation of the mask portion. The device thus constitutes a laryngeal mask. In practice, the annular peripheral formation has been made as an inflatable tube, e.g. of a silicone rubber.

This device has proved successful in use. Insertion of the device has been found to be easy and convenient in the majority of patients. A laryngoscope is not usually required. The mask does not enter the larynx or trachea so the risk of damage to these structures is avoided and the tracheal lumen is not narrowed as it is by insertion of an endotracheal tube. The risk of accidental entry into the oesophagus or one of the main bronchi is also avoided. Once in place the laryngeal mask generally permits the lungs to be ventilated by positive pressure. Alternatively the patient may be permitted to breathe spontaneously.

To avoid the risk that the epiglottis could obstruct the airway by falling inwards into the lumen of the mask and blocking the opening of the tube therein, which could happen, for example, with small displacements of the mask which may occur during surgery or manipulation of the patient on the operating table, I have described in my Patent Application No. 8713173 (Publication No 2205499A) an artificial airway device of the kind described above wherein the airway tube opens into the lumen of the mask through an aperture which is provided with means, such as flexible cross-bars, to prevent it from being obstructed by the epiglottis while permitting passage of a second smaller tube, when required. Such a tube may be, for example, an endotracheal or endobronchial tube or a suction catheter, or an inspection tube such as a fibre-optic broncho- or laryngoscope.

However, in the forms described in the above referenced documents the mask is secured to the airtube and so is suitable only for orotracheal intubation. The shape and size of the mask prevent its insertion through the nasal passages. Hence the problems associated with nasal intubation remain unaddressed.

According to the present invention, there is provided an artificial airway device to facilitate lung ventilation in an unconscious patient, comprising a mask having a flexible annular peripheral formation of roughly elliptical shape capable of conforming to, and of readily fitting within, the actual and potential space behind the larynx so as to form a seal around the circumference of the laryngeal inlet without the device penetrating into the interior of the larynx, the annular peripheral formation surrounding a hollow interior space or lumen of the mask into which opens an airway conduit whose length is of the same order as that of the mask; and an airway tube connectable to the airway conduit via detachable connecting means, so that the mask can be inserted into operative position in a patient via the mouth while the airway tube can subsequently be inserted through the nose and connected to the airway conduit by the detachable connecting means. Preferably the airway tube is sufficiently rigid to be usable for the insertion of the mask into the patient via the mouth when connected to the airway conduit by the detachable connecting means.

The airway conduit can have a flared end region for guiding and receiving the airway tube when it is inserted into a patient for connection to the mask.

Preferably the detachable connecting means comprises an inflatable cuff provided at one end region of the airway tube arranged so that, when inflated, it secures the airway tube within the end of the airway conduit. To improve the security of the connection the airway conduit can define a cavity for receiving the inflatable cuff.

Other types of detachable connecting means are envisaged, for example a twist/lock mechanism.

The flexible annular peripheral formation of the mask is preferably inflatable.

The artificial airway device according to the preferred embodiment of the invention can be used to carry out nasal intubation as follows. The end of the airway tube is inserted into the airway conduit and the cuff is inflated to secure the mask to the airway tube. The assembly then resembles the laryngeal mask for orotracheal intubation and can be inserted through the mouth in the manner described in the above-referenced British Patent No. 2111394B. The following steps are not possible with the device described in that patent and can only be carried out with the device of the present invention. Once the mask has been properly located, preferably by inflating the flexible annular peripheral formation, the inflatable cuff on the airway tube is deflated thereby releasing the airway tube from the mask and enabling it to be withdrawn through the mouth. It is then reinserted through the nose and guided again into the airway conduit via the nasal passages whereupon the inflatable cuff is again inflated to resecure the airway tube to the mask.

This device has the advantage of avoiding the need to paralyse the patient and also avoiding the particular difficulties of performing endotracheal intubation through the nose.

To improve the seal of the mask with the laryngeal inlet the annular peripheral formation can carry a soft, flexible, upstanding collar surrounding the lumen of the mask so as to improve the sealing contact with the tissues around the circumference of the laryngeal inlet.

Preferably the collar is formed of a flexible sheet material, and is adhered at its base to the adjacent surface of the annular peripheral formation.

In a preferred form of the mask, the inflatable peripheral formation is formed as a tubular ring and the collar is curved, as seen in cross-section, in the reverse sense to the walls of the tubular ring, so that the base of the collar is parallel to the adjacent surface of the ring and its free end extends away from the lumen of the mask.

The tube and collar may be made of a silicone rubber sheet material of similar thickness to one another.

Specific embodiments of the invention will now be described in more detail by way of example and with reference to the accompanying drawings, in which:-

Figure 1 is a side view of an artificial airway device, in the form of a laryngeal mask and an airway tube;

Figure 2 is a plan view of the mask with the periphery inflated;

Figure 3 is a section on the line III-III of Figure 2,

Figures 4 and 5 show diagrammatically the steps of using the device in a patient; and

Figures 6 and 7 show a device in which the airway conduit has a flared end.

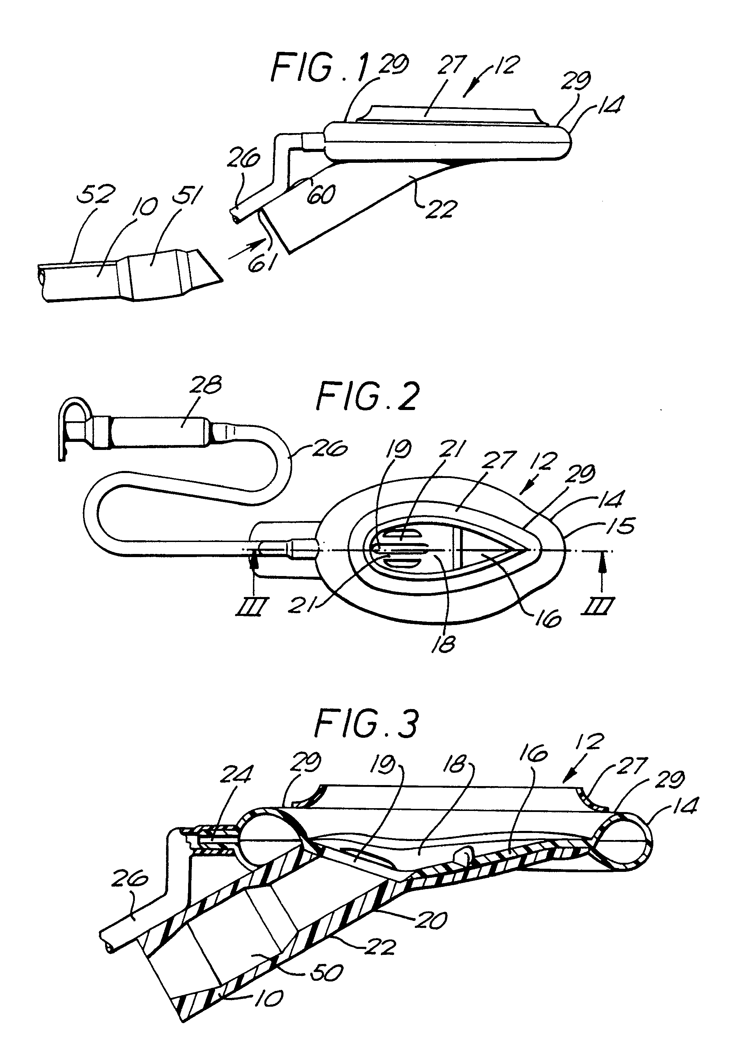

The artificial airway device illustrated in Figures 1 to 4 of the drawings comprises a laryngeal mask 12 of flexible silicone rubber sheet material, having an inflatable tubular ring 14 of the same silicone rubber material forming its periphery and a web 16 which closes off the rear of the interior or lumen 18 of the mask and is formed with an aperture 19. The mask has an airway conduit in the form of a short piece 22 of thick-walled silicone rubber tubing with one end moulded to fit against the outer edge of the web 16 and around the aperture 19, so as to form a semi-rigid backpiece for the mask, with the conduit 22 directed at an angle of substantially 30° to the plane of the ring 14. As can be seen most clearly in Figure 3, the airway conduit 22 defines a cavity 50. The device also includes a flexible airway tube 10 formed of small gauge plastic material, for example 7mm in diameter, carrying on one end an inflatable cuff 51, which can be inflated through an airline 52. The airway tube 10 can be connected to the conduit 22 by inserting the cuff 51 in the deflated state into the cavity 50 of the conduit 50 and then inflating the cuff 51 via the airline 52. There is so formed a secure connection between the airway tube 10 and the conduit 22. The airway tube 10 then opens into the interior or lumen 18 of the mask 12 through the piece 22 and the aperture 19.

The peripheral ring 14 is of roughly elliptical shape as seen in plan (Figure 2) though its distal end 15 may be slightly elongated to conform with the triangular shape of the base of the hypopharynx where it becomes continuous with the upper end of the oesophagus. The airway conduit 22 lies in substantially the same plane as the major axis of the peripheral ring 14 and at substantially 30° to the plane of the ring 14. The ring 14 is formed with a port 24 into which is sealed one end of a flexible silicone rubber tube 26 of much smaller diameter. The other end of tube 26 is provided with an inflation indicator 28, and can be connected to a small pump (not shown) such as a disposable 20 ml medical syringe for inflation of the ring 14. The tube 26 is shown fully in Figure 2 and in part only in Figures 1 and 3. Alternatively the tube 26 may be permanently connected through a valve to a collapsible bulb whose capacity is equal to the optimal inflated capacity of the ring 14. Similar options are available for inflation of the cuff 51 via the airline 52. Part of the tube 26 is secured to the conduit 22, e.g. by welding at zones 60,61 (Figure 1) to facilitate insertion and removal of the mask.

The aperture 19 through which the airway tube 10 opens into the lumen 18 of the mask 12 is provided with two flexible cross-bars 21 extending across the aperture 19 substantially parallel with the major axis of the peripheral ring 14, so as to leave the middle of the aperture clear for passage of an inspection or other tube. The bars 21 effectively prevent the epiglottis from falling into the aperture 19 and obstructing the airway.

A soft flexible upstanding collar 27, surrounding the lumen 18 of the mask, is formed of flexible sheet material, e.g. of a silicone rubber sheet material of similar thickness to that of the tubular ring 14. The collar 27 is adhered at its base to the adjacent surface 29 of the ring 14, and is curved, as seen in cross-section in Figure 3, in the reverse sense to the walls of the ring 14, so that the base of the collar 27 is parallel to the adjacent surface 29 of the ring 14 and its free end extends away from the lumen 18 of the mask. The collar 27 is designed to offer a low profile on deflation of the ring 14, to assist insertion and removal of the laryngeal mask. When the laryngeal mask is in place and the ring 14 is inflated, the collar 27 is found to improve the effectiveness of the seal between the mask and the tissues of the laryngeal inlet by about 30% on average, and thereby to reduce the risk of allowing air under positive pressure into the stomach and to improve the exclusion of any regurgitated food from the interior of the mask and hence from the larynx.

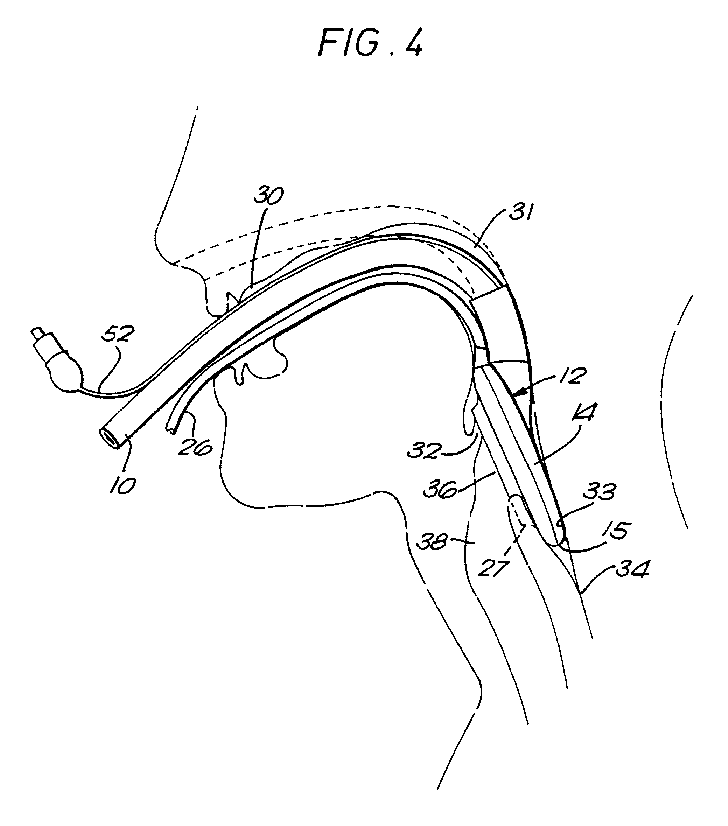

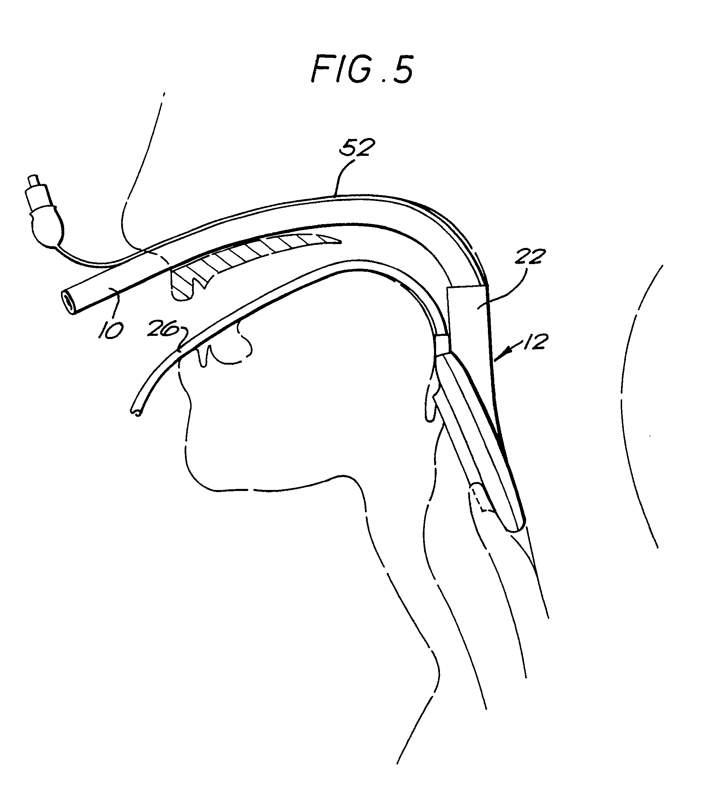

Different sizes of mask are needed for different sizes of patient. The use of the mask will now be described with reference to Figures 4 and 5. The ring 14 is first fully deflated, the airway tube 10 is secured to the airway conduit 22 as described above, and the device is inserted through the patient's mouth 30 and down through the throat 31 past the epiglottis 32 until the mask 12 comes to rest in the position shown in Figure 4, with the distal end 15 of the ring 14 in the base 33 of the throat, lying against the upper end of the normally closed oesophagus 34, which the mask cannot easily enter provided that the correct size has been chosen. The ring 14 is then inflated as shown to increase the sealing pressure around the inlet 36 to the larynx 38. The collar 27 is flattened between the ring 14 and the inlet 36 to improve the seal. The patient's airway is thus secure and unobstructed and the laryngeal mask will hold itself in place. The cuff 51 on the airway tube 10 is deflated and the tube 10 is then removed from the patient via the mouth and reinserted through the nose (Figure 5) until it enters the conduit 22. The cuff 51 is then reinflated to securely connect the airway tube 10 to the mask 12. The mask can then be connected via the airway tube directly to conventional anaesthetic circuit hosing for either positive pressure or spontaneous breathing. As can be seen from Fig. 5, the airway tube 10 opens into the lumen of the mask 12 at the appropriate angle (substantially 30°) to enable an inspection or other tube (not shown) passed through the airway tube 10 and the mask 12 to emerge at the correct angle for intubation of the larynx 38.

The embodiment described above may be used as a disposable instrument or as a re-usable one.

Although only a single collar 27 has been described above and shown in the accompanying drawings, it would be possible for the ring 14 to carry two or more such collars, disposed parallel to and one within the other.

The tube and mask portion could be made of other sterilisable materials, such as plastics. The materials may be more rigid than the inflatable silicone rubber materials described above. With some materials it may not be necessary that the peripheral ring should be inflatable. For example, the ring 14 may consist of a foam material within an air-tight covering, from which the air is evacuated to facilitate insertion of the mask. In any case the mask 12 will be shaped as described above to conform to and fit readily into the actual and potential space behind the larynx and to seal around the laryngeal inlet. The reference to actual and potential space will be understood to refer to the space normally available and that which can become available on flexure of the surrounding structures.

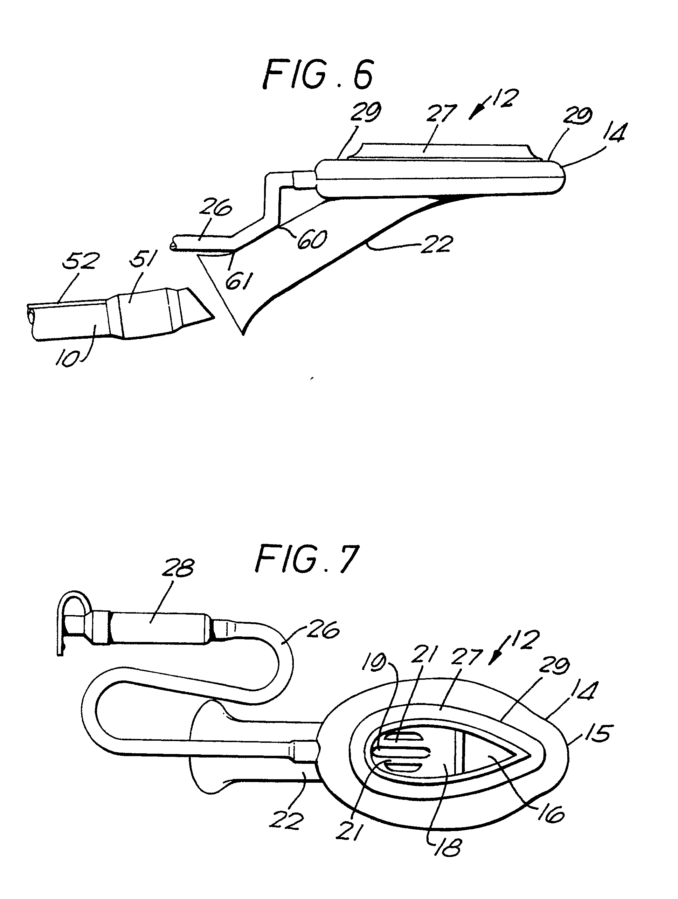

Figures 6 and 7 show a mask in which the end of the airway conduit 22 is flared to guide and receive the airway tube 10 so as to facilitate blind insertion.

## Claims

1. An artificial airway device to facilitate lung ventilation in an unconscious patient, comprising a mask having a flexible annular peripheral formation of roughly elliptical shape capable of conforming to, and of readily fitting within, the actual and potential space behind the larynx so as to form a seal around the circumference of the laryngeal inlet without the device penetrating into the interior of the larynx, the annular peripheral formation surrounding a hollow interior space or lumen of the mask into which opens an airway conduit whose length is of the same order as that of the mask; and an airway tube connectable to the airway conduit via detachable connecting means, so that the mask can be inserted into operative position in a patient via the mouth while the airway tube can subsequently be inserted through the nose and connected to the airway conduit by the detachable connecting means.

2. An artificial airway device according to claim 1 wherein the airway tube is sufficiently rigid to be usable for the insertion of the mask into the patient via the mouth, when connected to the airway conduit by the detachable connecting means.

3. An artificial airway device as claimed in claim 1 or 2 wherein the detachable connecting means comprises an inflatable cuff provided at one end region of the airway tube arranged so that, when inflated, it secures the airway tube within the end of the airway conduit.

4. An artificial airway device as claimed in claim 3 wherein the airway conduit defines a cavity for receiving the inflatable cuff.

5. An artificial airway device as claimed in claim 1, 2, 3 or 4 wherein the flexible annular peripheral formation of the mask is inflatable.

# FIG.1

# FIG.2

# FIG.3

FIG . 4

# FIG.5

## FIG.6

## FIG.7